# EUROPEAN PATENT SPECIFICATION

(11) **EP 2 580 183 B1**
(45) Date of publication and mention of the grant of the patent: **23.07.2014**
(21) Application number: 11725636.2
(22) Date of filing: 09.06.2011
(51) Int. Cl.: C07C 217/74, C07C 233/18, C07C 255/37

(54) **NEW PROCESS FOR THE PREPARATION OF N-[2-(7-METHOXY-1-NAPHTHYL)-ETHYL]ACETAMIDE**
NEUES VERFAHREN ZUR HERSTELLUNG VON N-[2-(7-METHOXY-1-NAPHTHYL)-ETHYL]ACETAMID
NOUVEAU PROCÉDÉ DE PRÉPARATION DE N-[2-(7-MÉTHOXY-1-NAPHTYL)-ÉTHYL]ACÉTAMIDE

(30) Priority: 10.06.2010 US 353266 P
(43) Date of publication of application: 17.04.2013
(73) Proprietor: GADOR S.A., Buenos Aires 1414 (AR); Conicet -Consejo Nac. De Investigaciones Científicas Y Técnicas, C1033 Buenos Aires (AR)
(72) Inventor: TOMBARI, Dora, Graciela, 1772 Villa Celina Buenos Aires (AR); MANGONE, Constanza, Pia, 1425 Ciudad Autonoma de Buenos Aires (AR); GARCIA, Maria, Beatriz, 1636 Olivos Buenos Aires (AR); VECCHIOLI, Adriana, C1126 Ciudad Autonoma de Buenos Aires (AR); LABRIOLA, Rafael, Alberto, B1643 Beccar (AR); BURTON, Gerardo, B1602 Florida Buenos Aires (AR); RUSTOY, Eduardo, Miguel, 6700 Lujan Buenos Aires (AR)
(74) Representative: Goddar, Heinz J.
(86) International application number: PCT/EP2011/002834
(87) International publication number: WO 2011/154140

(56) References cited:
- EP-A1- 1 564 202
- WO-A1-96/08466
- FOURMAINTRAUX E ET AL: "TETRAHYDRONAPHTHALENIC DERIVATIVES AS NEW AGONIST AND ANTAGONIST LIGANDS FOR MELATONIN RECEPTORS", BIOORGANIC & MEDICINAL CHEMISTRY, PERGAMON, GB, vol. 6, no. 1, 1 January 1998 (1998-01-01) , pages 9-13, XP002074450, ISSN: 0968-0896, DOI: 10.1016/S0968-0896(97)00175-2

## Description

### FIELD OF THE INVENTION

The present invention refers to a novel process for the preparation of N-[2-(7-methoxy-1-naphthyl)ethyl]acetamide, also known as agomelatine, Compound (I).

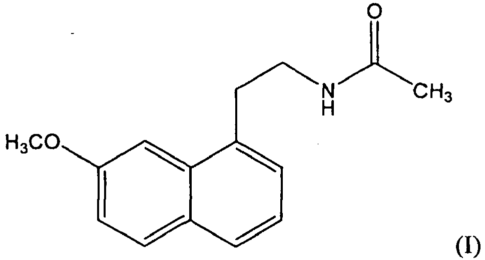

### BACKGROUND OF THE INVENTION

The compound N-[2-(7-methoxy-1-naphthyl)ethyl]acetamide, also known as agomelatine (trade names Valdoxan, Melitor, Thymanax), presents hypnotic and antidepressant properties which make it suitable for clinical use (Pharmaprojects Access No. 19325 (27-1-2010)).

This compound was first described in EP 0447285 B1 (1991) and US 5225442 A (1992). It's preparation has been described, in addition to the aforementioned patents, in S. Yous, et al. J. Med. Chem. 35, 1484 (1992), as well as EP 1564202 B1 (2005), EP 1564203 B1 (2005), EP 1564204 B1 (2005), EP 1564205 B1 (2005), US 7470806 B2 (2005), US 7479569 B2 (2005), US 7476751 B2 (2005), US 2010/0036161 A1 (2009), US 2010/0036163 A1 (2009), and US 2010/0036162 A1 (2009).

In addition, various crystalline forms of agomelatine such as forms II, III, IV, V and VI of agomelatine have been described in documents EP 1564202 B1 (2005), US 7250531 B2 (2005), EP 2008993 A1 (2006), US 7635721 B2 (2006), US 7476751 B2 (2005), US 7498465 (2007), and US 7498466 B2 (2007). AR 047741 B1 (2005) claims the crystalline form II of agomelatine. Other patent documents such as AR 057713 (2006), AR 057714 (2006) and AR 057715 (2006) claim the crystalline forms III, IV and V of agomelatine. AR 47667 (2006) and AR 48235 (2006) also claim the intermediate synthesis products.

Recently, crystalline forms A, B, and C have been described in WO 2011/006387 A1 (2010).

As mentioned in the literature, the synthetic pathway disclosed in United States Patent US 5225442 A, using 7-methoxy-1-tetralone as starting material, was improved over in simplicity, economy, and purity of yield by the industrial synthesis process described in patents EP 1564203 B1, EP 1564204 B1, and US 7479569 B2.

Later, another synthetic process was described in document WO 2009/053545 A2 (2008), using 7-methoxy-1-naphthoic acid as starting material. This process, significantly longer than the previous ones, implied a further reduction in production costs due to the even lower cost of the new starting material used.

However, there is still a need for a synthetic process for the active ingredient agomelatine which not only provides improved economic performance but also simplifies the procedure without the need for special equipment, as in the case of high pressure hydrogenation, and without the need to isolate intermediate products for further purification.

### SUMMARY OF THE INVENTION

The present invention concerns a novel process using 7-methoxy-tetralone as starting material for the preparation of N-[2-(7-methoxy-1-naphthyl)ethyl]acetamide, also known as agomelatine, as claimed in claims 1-19.

In a first embodiment of the invention, 7-methoxy-tetralone is processed in accordance with Scheme I to form agomelatine. ,

In a second embodiment of the invention, 7-methoxy-tetralone is processed in accordance with Scheme II to form agomelatine.

Disclosed, but not being part of the invention is also a crystalline form X of agomelatine and a process for its preparation.

### BRIEF DESCRIPTION OF THE DRAWINGS

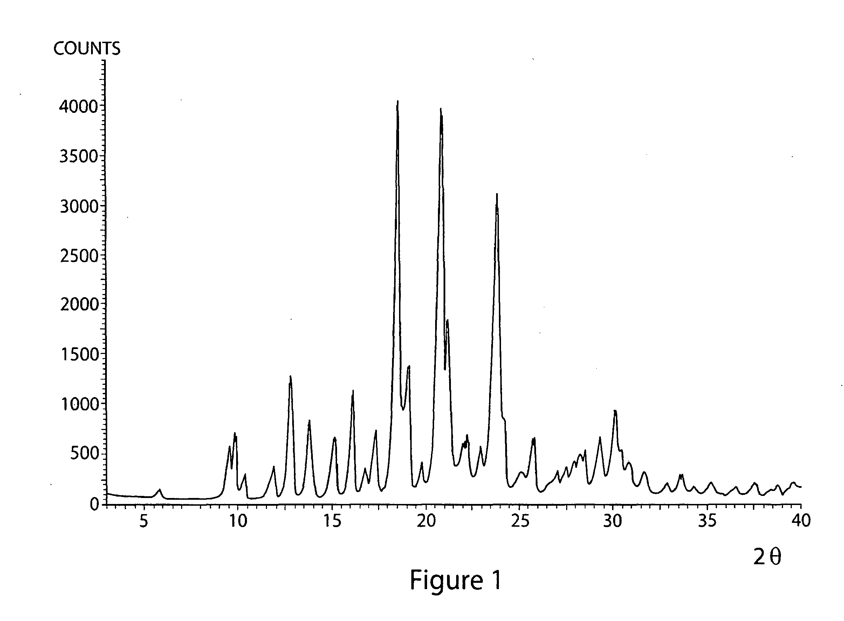

- Fig. 1: shows the XRPD of Agomelatine Form X,

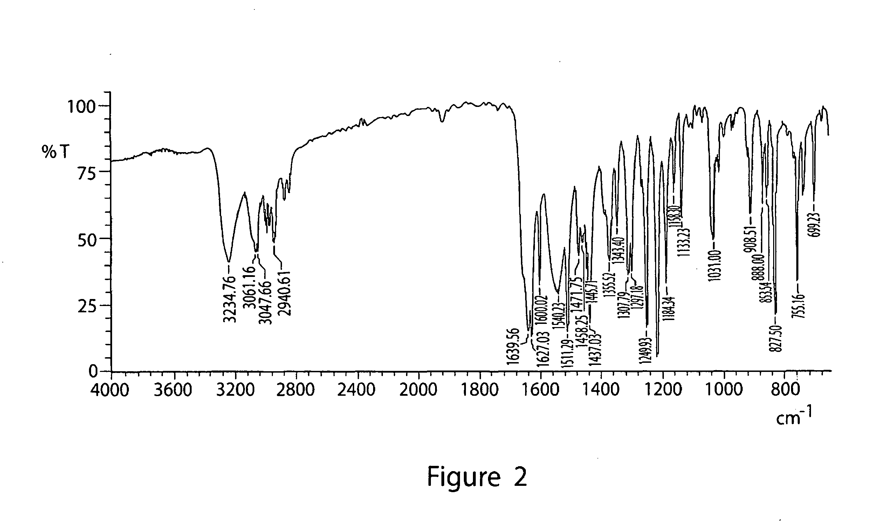

- Fig. 2: shows the IR of Agomelatine Form X,

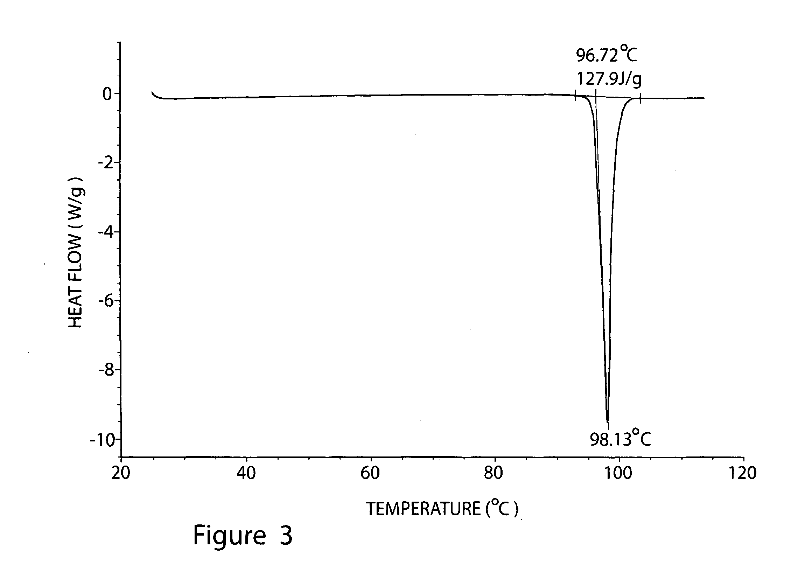

- Fig. 3: shows the DSC of Agomelatine Form X.

### DETAILED DESCRIPTION OF THE INVENTION

The novel synthesis starts with 7-methoxy-1-tetralone.

In accordance with the first embodiment as shown in Scheme I, the first reaction of Scheme I comprises a condensation between the anion from a cyanomethyl phosphonic acid ester in the presence of a base such as NaH, LiOH, potassium *tert-*butoxide with 7-methoxy-1-tetralone to produce 7-methoxy-1-tetrahydro naphthalene acrylonitrile.

The phosphonic ester is a commercially available substance; it is created according to the usual techniques from bromine acetonitrile and triethyl phosphite.

To use in similar cases, please refer to (S. Durieux et al - Bioorganic & Medicinal Chemistry 17, 2963 (2009).

This condensation produces the exclusively exocyclic double bond substituted acrylonitrile, as it is shown by its NMR 1H spectrum, which simplifies its purification.

The double bond substituents geometry is not important, since the product will be reduced in the following step.

Through the use of metallic hydrides in the presence of salts, especially NaBH₄/NiCl₂, the double bond reduction is concomitant to that of the nitrile group.

In this way, a substituted naphthyl-ethylamine is obtained, the purification of which is very simple due to its basic properties.

The pure good yield Agomelatine is easily obtained through acetylation and subsequent aromatization.

For the aromatization, 2,3-dichloro-5,6-dicyano-1,4-benzoquinone (DDQ) is used, which does not require precious metals catalysts.

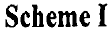

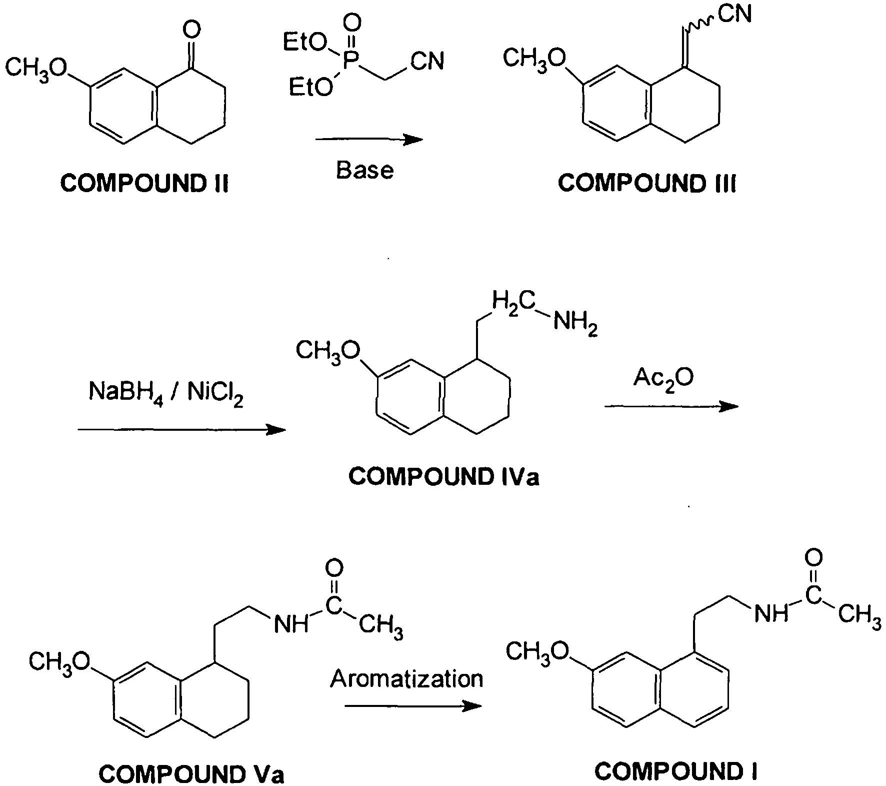

The synthesis according to Scheme I has the following differences and advantages over the industrial synthesis process described in patents EP 1564203 B1, EP 1564204 B1, and US 7479569 B2, which renders it useful as a synthesis alternative:
- The intermediate products for both processes are different.
- In the condensation step of Scheme I, the presence of ammonium salts from relatively long side chain acids as catalyst is not necessary; only the presence of a base is necessary.
- In the synthesis of 7-methoxy-1-tetrahydronaphtalene-acrylonitrile, only the product with double exocyclic bond is obtained; the geometric isomerism is not important since the product will be reduced.
- The reduction of the nitrile and of the double bond is simultaneous.
- The reduction shown in Scheme I is done with an economic and simple reagent, without the need for special equipment, as in the case of a high pressure hydrogenation.
- The acylation is done using an intermediate product neither protected by nor used in patents EP 1564203 B1, EP 1564204 B1, and US 7479569 B2.
- The final aromatization step is done by oxidation with DDQ, producing a clean and good yield product; the raw product obtained is purified by recrystallization in different solvents such as methanol, ethyl acetate, toluene, acetonitrile, ethanol, isopropanol, or mixtures thereof or with water.

As regards the product obtained by Scheme I, its physical parameters (fusion point and NMR 1H spectrum) relate to those described in documents J. Med. Chem. 35, 1484 (1992), EP 447285 B1 (1991) and US 5225442 A (1993).

In the second embodiment of the invention N-[2-(7-methoxy-1-naphthyl)ethyl]acetamide, also known as agomelatine, Compound (I), is produced according to Scheme II below:

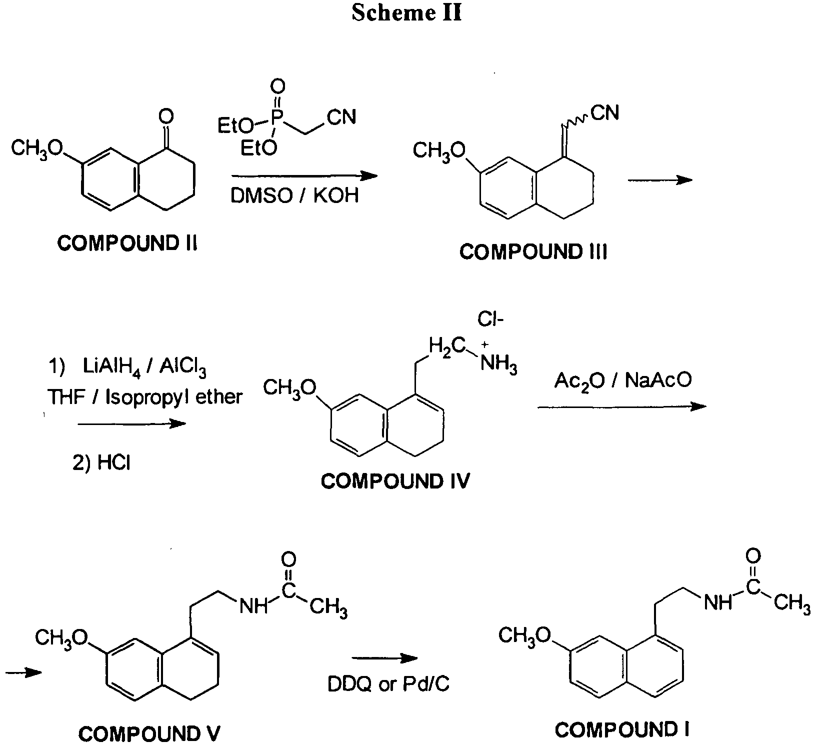

In this embodiment, the inventors developed a new process for the industrial synthesis of agomelatine, which is simplified and highly reproducible, and which provides high yield and does not involve the need to carry out troublesome isolation and purification procedures on intermediate compounds of the synthesis.

More specifically, the Scheme II involves a procedure for the synthesis of N-[2-(7-methoxy-1-naphthalenyl)ethyl]acetamide, Compound (I), comprising, in a first step, the condensation of 7-methoxy-tetralone, Compound (II), with an ester of the cyanomethylphosphonic acid ((RO)₂P(O)CH₂CN, wherein R= alkyl, aryl, alkyl aryl) in solution into an ether solvent, such as di-isopropyl ether, tetrahydrofuran, or dimethyl sulfoxide, preferably dimethyl sulfoxide (DMSO), and the addition of a base, such as NaH, Li₂O, LiOH, NaOH, KOH or potassium *tert*-butoxide, preferably potassium hydroxide (KOH), to give (7-methoxy-3,4-dihydro-2H-naphthalene-1-ylidene)-acetonitrile, Compound (III).

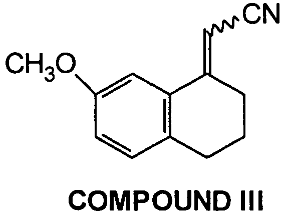

Compound (III), (7-methoxy-3,4-dihydro-2H-naphthalene-1-ylidene)-acetonitrile, is then combined with metal hydrides in the presence of Lewis acids in an aprotic medium to give compound 2-(7-methoxy-3,4-dihydro-2H-naphthalene-ylidene)-ethylamine, which, without further isolation and through the action of an acid, isomerizes to a salt of 2-(7-methoxy-3,4-dihydro-naphthalene-1-yl)-ethylamine, Compound (IV), wherein the double bond is endocyclic.

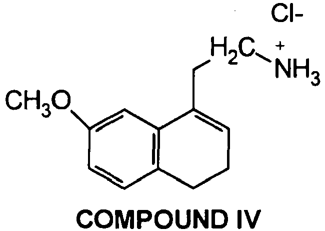

The 2-(7-methoxy-3,4-dihydro-naphthalene-1-yl)-ethylamine salt is then acetylated with an acetylating agent in order to produce the corresponding acetamide, Compound (V), wherein the endocyclic double bond is maintained.

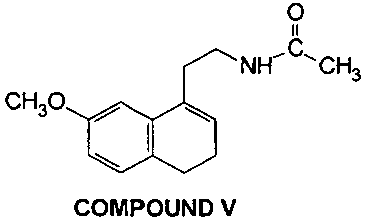

Finally, in a fourth and last step, Compound (V) is oxidized to give Compound (I), named agomelatine, or N-[2-(7-methoxy-1-naphthyl)ethyl]acetamide.

In a preferred aspect of the present invention for the obtention of Compound (IV), the pharmaceutically acceptable metal hydride used in the procedure of the invention is NaBH₄ or LiAlH₄, preferably LiAlH₄.

Pharmaceutically acceptable Lewis acids used for the obtention of Compound (IV) in the invention include AlCl₃, NiCl₂, or CoCl₂. Preferably, AlCl₃ is the pharmaceutically acceptable Lewis acid of choice in the present invention.

Also preferred in the present invention for the obtention of Compound (IV) is the use of a pharmaceutically acceptable aprotic solvent, such as an ether compound, selected from the group consisting of di-isopropyl ether, tetrahydrofuran (THF), or mixtures thereof with toluene.

Also, the pharmaceutically acceptable acid used for the isomerization and obtention of the salt of compound (IV) is an aqueous acid selected from the group consisting of HCl, H₂SO₄, or HBr, preferably HCl.

In another particular aspect of the present invention, for the obtention of Compound (V), the pharmaceutically acceptable acylating agent used in the procedure of the invention is acetic anhydride/sodium acetate.

In another particular aspect of the present invention, for the obtention of Compound (V), the pharmaceutically acceptable acylating agent used in the procedure of the invention is acetyl chloride in the presence or absence of a base such as triethylamine, pyridine, potassium carbonate, sodium acetate, or the like.

In a further particular aspect of the present invention, for the obtention of Compound (I), the pharmaceutically acceptable oxidizing agent used in the procedure of the invention is 2,3-dichloro-5,6-dicyanobenzoquinone (DDQ).

In a further preferred aspect of the present invention, for the obtention of Compound (I), oxidation takes place through catalysis with Pd/C, without further addition of co-catalyzers, in hydrocarbonated alkyl or aryl solvents.

The invention also refers to a procedure for the purification of Compound (I), obtained according to the procedure described herein, comprising a crystallization process wherein the crystallization solvent is selected from the group consisting of an alcohol of up to 4 carbon atoms, ethyl acetate, methyl-ethyl-ketone, acetone, isopropyl ether, toluene, acetonitrile, or mixtures thereof, with the possible addition of water.

The invention also refers to Compound (I), obtained according to the procedure described herein, and whose physical parameters, as well as its melting point, correspond to those described in prior art documents.

Finally, the Applicant has now developed a new synthesis process that allows agomelatine to be obtained in a well defined reproducible new crystalline form, which especially exhibits valuable characteristics for formulations, but is not part of the invention.

Also disclosed is the crystalline form X of the Compound (I), characterized by the following X-ray Powder Diffraction Diagram, measured using a Philips model X'Pert PW3710 (Philips, The Netherlands) with a wavelength of 1.54060 (K-Alpha) and expressed in terms of inter-planar distance d, Bragg's angle 2 theta, intensity and relative intensity (expressed as a percentage of the most intense ray).

| 2-Theta (°) | d (Å) | Intensity (%) |
|---|---|---|
| 5.79 | 15.251 | 1.94 |
| 9.56 | 9.246 | 12.64 |
| 9.85 | 8.969 | 15.46 |
| 11.90 | 7.432 | 8.48 |
| 12.81 | 6.905 | 30.54 |
| 13.83 | 6.399 | 18.75 |
| 15.14 | 5.846 | 14.58 |
| 16.14 | 5.487 | 27.13 |
| 17.33 | 5.114 | 16.45 |
| 18.56 | 4.776 | 100.00 |
| 19.10 | 4.644 | 33.0 |
| 19.80 | 4.480 | 9.45 |
| 20.84 | 4.258 | 98.52 |
| 21.18 | 4.192 | 44.12 |
| 22.02 | 4.034 | 13.59 |
| 22.24 | 3.994 | 15.31 |
| 22.94 | 3.874 | 13.41 |
| 23.82 | 3.733 | 77.39 |
| 24.23 | 3.671 | 18.55 |
| 25.70 | 3.463 | 15.12 |
| 25.79 | 3.451 | 13.53 |
| 27.91 | 3.195 | 9.28 |
| 28.28 | 3.154 | 12.11 |
| 28.52 | 3.127 | 12.67 |
| 29.28 | 3.047 | 14.28 |
| 30.10 | 2.966 | 22.47 |
| 30.46 | 2.932 | 12.24 |
| 30.84 | 2.897 | 9.37 |

This crystalline form is also characterized by its IR spectrum (KBr) presenting characteristic absorption signals at 3235, 3061, 3048, 2941, 1640, 1627, 1600, 1540, 1511, 1437, 1308, 1297, 1216, 1184, 1031, 827, 755 cm⁻¹ (FT-IR Shimadzu IRAffinity/1).

The crystalline form X is also characterized by DSC (Differential Scanning Calorimetry) (Q-100 TA INSTRUMENTS equipment at 10°C/min).

Also disclosed is a pharmaceutical composition comprising, as an active ingredient, Compound (I) and the crystalline form X of compound (I), obtained according to the procedure described herein, and one or more pharmaceutically acceptable excipients, such as those described in the Handbook of Pharmaceutical Excipients, 2nd Edition, American Pharmaceutical Association; The Theory & Practice of Industrial Pharmacy, 2nd Edition, Lachman Leon, 1976; Pharmaceutical Dosage Forms: Tablets, Volume 1, 2nd Edition, Lieberman, Hebert A, et al, 1989; Modern Pharmaceutics. Banker, Gilbert and Rhodes, Christopher T, 1979; and Remington's Pharmaceutical Sciences, 15th Edition, 1975, for their use in the preparation of a medicament for the treatment of depression and sleep disorders, among others.

Also disclosed is a process for the preparation of the crystalline form X of Agomelatine, which process is characterised in that Agomelatine is dissolved into acetone and the obtained solution is poured into a cool mixture of acetone:water.

### Examples

After having described the method for the obtention of agomelatine, there follow preparation examples for illustrative purposes only, which aim at showing the processes involved and the possibilities thereto.

### Example 1: 7-Methoxy-1,2,3,4-tetrahydro naphthylidene acetonitrile, Compound III (Scheme I)

In a 2 L capacity three necked round flask, placed in a water bath and provided with condenser, thermometer, mechanic stirrer, addition funnel and nitrogen flow, sodium hydride (17.81 g - 60% w/w) and tetrahydrofuran (135 ml) are added. A soft nitrogen flow is kept while diethyl cyanomethyl phosphonate (80 g) in tetrahydrofuran (70 ml) is slowly added at room temperature. Gas release is observed together with a slight exothermic reaction. During the addition, the internal temperature is kept between 20-30°C. A slightly colored suspension is obtained.

Once the addition of the phosphonate solution is finished, the addition of a 7-Methoxytetralone (43 g) in tetrahydrofuran (80 ml) solution is started, keeping internal temperature between 30-35°C.

The suspension is stirred at room temperature for 1-2 hours and controlled by TLC (mobile phase: toluene (Rf 7-MT: 0.3; Rf product 0.35; Rf phosphonate: 0)).

Once the reaction is finished, it is cooled at 0-5°C and water is added (270 ml). Ethyl acetate (1 x 270 ml and 2 x 135 ml) is added and the organic layers are combined.

The solvent is evaporated until just reaching dryness at reduced pressure, thereby obtaining a viscous oil (58 g) (Titration: 75%) Yield: 90%.

This oil can be used directly in the following step without further purification.

A sample of the oil is treated with methanol, placed in an ice bath and obtaining a crystallized solid which, after filtration and drying, shows a purity above 92% (HPLC) and is characterized by spectroscopy.

NMR ¹H: (δ) 2.87 or 2.58 (E/Z) (2H, dt, J=1.6 and 6.4 Hz) (CH₂ allyl); 5.70 and 5.27 (E/Z) (2H, t, J= 1.5 and 1.4 Hz); 7.03 or 7.86 (E/Z) (1H, d, J=2.8 or 2.6 Hz) (H-8).

### Example 2: 1-(2-aminoethyl)-7-methoxy-1,2,3,4-tetrahydro naphthalene, Compound IVa (Scheme I)

The product obtained in the previous step is dissolved (1.2 g containing approximately 0.8 g of 7-Methoxy-1,2,3,4-tetrahydro naphthylidene acetonitrile) in methanol (10 ml) and anhydrous NiCl₂ (0.52 g, 4 mmol) is added.

Then, NaBH₄ (0.83 g, 22 mmol) is added while stirring and cooling (exothermic reaction with plenty of gas release).

The black colored reaction mixture is stirred for 12 hours at room temperature until the starting material is disappeared by TLC.

The solution is filtered through celite, and the cake is washed with three portions of 50 ml ethyl acetate (AcOEt).

The solvent is evaporated, water is added (20 ml), and the solution is extracted with three portions of AcOEt.

The organic phases are combined, dried over Na₂SO₄, filtered and evaporated to dryness.

The residue obtained is suspended in 15 ml concentrated HCl; the suspension is stirred at room temperature for 30 minutes and then it is extracted with three fractions of 10 ml AcOEt, and the organic phase is washed with concentrated HCl (2 x 10 ml).

The acid phases are combined and concentrated NaOH aqueous solution (16 g in 50 ml, final pH > 12) is slowly added in an ice bath until alkaline. The aqueous phase is extracted with AcOEt (4 x 20 ml), the organic phase is dried over Na₂SO₄ and the solvent is evaporated.

The result is 0.82 g of product containing approximately 85% amine (calculated by NMR ¹H). (Yield: 85 %).

NMR ¹H: (δ) 2.82 (2H, t, J= 5.6 Hz) (alpha H to amine group); 2.82 (1H, overlaying m) (benzyl H); 6.71 (1H, d, J= 2.6 Hz) (H-8).

### Example 3: 1-(2-N-acethyl aminoethyl)-7-methoxy-1,2,3,4-tetrahydro naphthalene, Compound Va (Scheme I)

The mass obtained in the previous step (containing approximately 0.7 g amine) is dissolved in 3 ml methanol and 1.5 ml acetic anhydride, and is stirred for 12 hours under reflux (Rf acetamide 0.1; mobile phase AcOEt/hexane 1:2).

The solution is evaporated under reduced pressure to eliminate the acetic acid. Methanol is added and the solution is evaporated to dryness.

It is obtained 0.82 g of product containing approximately 0.76 g amine calculated by NMR ¹H. (Yield: 90 %).

NMR ¹H: (δ) 1.95 (3H, s) (CH₃ CO); 3.2-3.5 (2H, m) (alpha H to NH); 2.80 (1H, tt, J= 9.2 and 5.0 Hz) (benzyl H); 3.76 (3H, s) (CH₃O); 6.7 (1H, d, J=2.6 Hz) (H-8).

### Example 4: Agomelatine, Compound I (Scheme I)

The acetylated product (0.82 g containing approximately 0.76 g amide) is dissolved in 10 ml anhydrous tetrahydrofuran (THF) and 9 mMol DDQ are added. The solution is stirred and heated under reflux for 12 hours until the starting material disappeared (TLC: Rf GML: 0.12, Rf acetamide: 0.1, mobile phase AcOEt/hexane 1:2).

The solvent is evaporated, 10 ml of a sodium bisulfite solution (2 g in 10 ml distilled water) are added, and the solution is stirred at 40°C for 30 minutes.

Then, 20 ml of a NaOH 1.25M aqueous solution are added until alkaline. The product is extracted with three portions of 30 ml AcOEt, and the solvent is evaporated resulting in a yellow solid (0.4 g). (Yield: 53 %).

A sample of this solid (0.1 g) is recrystallized in a methanol:water mixture, obtaining a melting point of 108-109 °C.

The NMR ¹H spectrum coincides with that of a standard Agomelatine.

Part of this solid (0.1 g) is dissolved in hot methanol, charcoal is added. Then it is hot filtered through a celite bed, and is crystallized by the water adding. It is left in the refrigerator overnight.

The solid (0.08 g) is dried for 3 hours at 50 °C and 133 Pa (1 Torr. MP): 108-109 °C. (EP 447285 B1 Ex.1 provides melting point: 109-110 °C).

### Example 5: (7-Methoxy-3,4-dihydro-2H-naphthalene-1-ylidene)-acetonitrile, Compound (III) (Scheme II)

Dimethyl sulfoxide (6.4 l) is placed into a 50-liter reactor and potassium hydroxide (1.28 kg; 22.85 M) is added.

A solution of cyanomethyl diethyl phosphonate (3.4 l; 21.03 M) in tetrahydrofuran (3.4 l) is added to the suspension and kept under stirring for 1 hour at 15 - 25°C.

Then, a solution of 7-methoxy-1-tetralone (2 kg; 11.4 M) in tetrahydrofuran (3.8 1) is added, keeping the temperature between 15 - 25°C. The suspension is stirred for 2½ hours.

Water (15 1) is added, while the temperature is maintained between 15 - 25°C.

Then, the reaction mixture is distilled under reduced pressure at a temperature lower than 50°C until the tetrahydrofuran is removed.

The reaction mixture is cooled at room temperature and extracted with toluene (3 x 10 l).

The combined organic phase is washed with sodium chloride aqueous solution 35% (w/v), and the organic phase is concentrated at reduced pressure at 60°C up to a final volume of approximately 4-5 l.

This solution contains around 2.15 kg (95%) (analyzed by HPLC) of (7-methoxy-3,4-dihydro-2H-naphthalene-1-ylidene)-acetonitrile, Compound (III), which is used as it is in the next step.

### Example 6: 2-(7-Methoxy-3,4-dihydro-naphthalene-1-yl)-ethylamine Hydrochloride, Compound (IV) (Scheme II)

A suspension of LiAlH₄ (107 g) in tetrahydrofuran (2200 ml) is placed in a 3-necked, 12-liter flask, equipped with stirrer, condenser and mild nitrogen flow.

AlCl₃ (240 g) in tetrahydrofuran (2200 ml) is added to the suspension.

After that, a solution of (7-methoxy-3,4-dihydro-2H-naphthalene-1-ylidene)-acetonitrile, Compound (III) (440 g with an 80% titration, equivalent to 352 g) in toluene (2000 ml) is added under stirring at room temperature. A slightly colored suspension is obtained.

After 4 hours, methanol (880 ml) and then water (880 ml) are added.

The suspension obtained is filtered, and the insoluble material is washed with methanol. The methanolic filtrates are treated with concentrated hydrochloric acid (HCl) (around 150 ml) until pH less than 1.

The acid solution is distilled at atmospheric pressure up to a volume of 1100 ml and then heated under reflux (80°C) for 1 hour.

Toluene (1320 ml) is added, and the suspension is cooled to 0-5°C.

The solid is isolated by filtration and dried at 60°C.

This procedure yields 371 g (88%) of 2-(7-methoxy-3,4-dihydro-naphthalene-1-yl)-ethylamine hydrochloride, Compound (IV), whose ¹H-NMR is coincident with that of a standard.

Its retention time in a chromatogram (HPLC) is coincident with that of a standard, and its purity is higher than 97.5%.

### Example 7: N-[2-(7-Methoxy-3,4-dihydro-naphthalene-1-yl)-ethyl]-acetamide, Compound (V) (Scheme II)

2-(7-Methoxy-3,4-dihydro-naphthalene-1-yl)-ethylamine hydrochloride, Compound (IV) (300 g, 1.25 moles) is placed in a 3-necked, 3-liter flask, equipped with stirrer, condenser, addition funnel and with mild nitrogen flow. Toluene (1200 ml) is added to obtain a suspension. After that, anhydrous sodium acetate (108 g, 1.31 moles) is added.

Then acetic anhydride (124.6 ml, 1.31 moles) is added at 20-25°C.

After 1 hour at room temperature, the suspension is filtered and the solid is washed with toluene.

Sodium hydroxide aqueous solution 10% w/v (1200 ml) is added to the filtrates.

The phases are separated, and the organic phase is washed with water until pH 6.

The toluene phase is concentrated at reduced pressure to a final volume of about 900 ml and hexane is added.

The suspension is cooled below 5°C for 1 hour; the solid is filtered, washed, and dried at 60°C.

257 g (84%) of N-[2-(7-methoxy-3,4-dihydro-naphthalene-1-yl)-ethyl]-acetamide, Compound (V) is obtained, which is used as it is for the final step.

### Example 8: N-[2-(7-Methoxy-1-naphthyl)ethyl]acetamide (agomelatine) (Scheme II)

To a solution of N-[2-(7-methoxy-3,4-dihydro-naphthalene-1-yl)-ethyl]-acetamide, Compound (V) (250 g, 1.02 moles) in tetrahydrofuran (2 1), DDQ (255 g, 1.12 moles) is added.

During the addition the internal temperature is kept around 20°C.

The reaction mixture is distilled at reduced pressure, replacing the solvent by toluene.

The final suspension is filtered at room temperature, and the obtained solid is suspended again in toluene at 40-45°C.

The combined toluene solution is distilled at reduced pressure to a final volume of about 250 ml.

The resulting suspension is cooled at 0-5°C and then filtered and dried in an oven at 60°C.

The solid obtained, whose ¹H-NMR spectrum coincides with that of a purified sample, is dried at 60°C.

Yield: 198 g (80%). Titration (HPLC): > 98%.

### Example 9: N-[2-(7-Methoxy-1-naphthyl)ethyl]acetamide (agomelatine) (Scheme II)

A suspension of N-[2-(7-methoxy-3,4-dihydro-naphthalene-1-yl)-ethyl]-acetamide, Compound (V) (200 g, 0.82 moles) and 10% Pd/C (10.2 g, 10 mmol) in toluene is heated under reflux until the starting product disappears.

The reaction mixture is filtered to separate the catalyzer, and the solution is distilled to a final volume of about 200 ml.

This suspension is cooled at 0-5°C for one hour and then filtered. The solid is dried in an oven at 60°C.

159 g (80%) of agomelatine, similar quality of that in Example 8 is obtained.

### Example 10: Crystalline form X

Agomelatine (6 kg) was dissolved into acetone (18 l) under reflux and the solution was cooled at 35-40 °C.

This solution was transferred to a second reactor vessel containing a mixture of acetone (12 l): water (60 l) at 0 °C.

The solution of Agomelatine was then poured under stirring into this mixture; a suspension was immediately observed.

This suspension was kept for 30 minutes at 0-5 °C.

The crystalline solid was filtered and dried at 60 °C in an oven until constant weight. Yield: 90 %.

## Claims

1. A procedure for the preparation of N-[2-(7-methoxy-1-naphthyl)ethyl]acetamide, Compound (I) 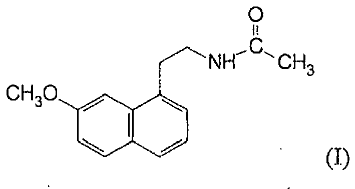 **characterized in that** Compound (II) is reacted with an ester of the cyanomethylphosphonic acid [(RO)₂P(O)CH₂CN, wherein R= alkyl, aryl, alkyl aryl] in an anhydrous basic medium to yield Compound (III); 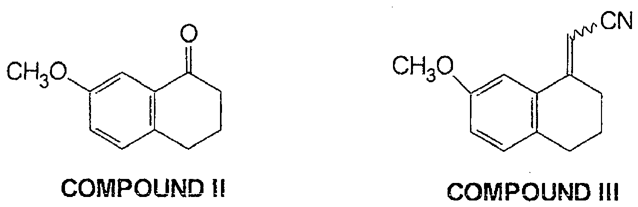 the compound (III) is then transformed into compound (IV), or a pharmaceutically acceptable salt thereof, through reduction with a metal salt of a metal hydride in an aprotic solvent, and its subsequent isomerization with a protic acid; 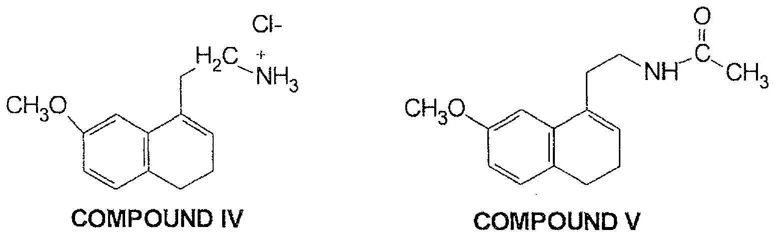 this reaction is followed by an acylation process to obtain Compound (V); and finally by oxidation (aromatization) of Compound (V) to obtain N-[(7-methoxy-1-naphthyl)ethyl]acetamide, Compound (I).

2. A procedure according to claim 1, wherein the base for the obtention of Compound (III) is NaH, Li₂O, LiOH, NaOH, KOH, or potassium *tert*-butoxide, preferably potassium hydroxide (KOH).

3. A procedure according to claim 1, wherein the solvent for the obtention of Compound (III) is an ether compound, such as di-isopropyl ether, tetrahydrofuran, or dimethyl sulfoxide, preferably dimethyl sulfoxide (DMSO).

4. A procedure according to claim 1, **characterized in that** the metal hydride used for the obtention of Compound (IV) is NaBH₄ or LiAlH₄, preferably LiAlH₄.

5. A procedure according to claim 1 and 4, **characterized in that** the metal hydride used in the presence of a Lewis acid is selected from the group consisting of AlCl₃, NiCl₂, or CoCl₂, preferably AlCl₃.

6. A procedure according to claim 1, **characterized in that** the aprotic solvent used for reduction with the metal hydride is an ether compound such as di-isopropyl ether, tetrahydrofuran, or mixtures thereof with toluene.

7. A procedure according to claim 1, **characterized in that** the protic acid used for the isomerization and obtention of Compound (IV) is an aqueous acid such as HCl, H2SO₄, or HBr, preferably HCl.

8. A procedure according to claim 1, **characterized in that** the acylating agent used for the obtention of Compound (V) is acetic anhydride/sodium acetate.

9. A procedure according to claim 1, **characterized in that** the acylating agent employed for the obtention of Compound (V) is acetyl chloride in the presence or absence of a base such as triethylamine, pyridine, potassium carbonate, sodium acetate, or the like.

10. A procedure according to claim 1, **characterized in that** the oxidizing agent in the final step of aromatization for the obtention of N-[2-(7-methoxy-1-naphthyl)ethyl]acetamide, Compound (I), is 2,3-dichloro-5,6-dicyanobenzoquinone (DDQ).

11. A procedure according to claim 1, **characterized in that** the final oxidation for the obtention of N-[2-(7-methoxy-1-naphthyl)ethyl]acetamide, Compound (I), is carried out through catalysis with Pd/C, without further addition of co-catalyzers, in an alkyl or aryl hydrocarbon.

12. A procedure for the purification of Compound (I), according to claim 1, wherein the crystallization solvent is an alcohol of up to 4 carbon atoms, ethyl acetate, methyl-ethyl-ketone, acetone, isopropyl ether, toluene, acetonitrile, or mixtures thereof, with the optional addition of water.

13. A procedure to prepare N-[(7-methoxy-1-naphtyl) ethyl] acetamide (Agomelatine) (Compound I) comprising:
(a) reacting compound II 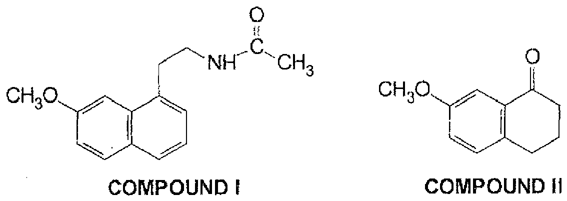 with (RO)₂P(O)CH₂CN, where R= alkyl, aryl, alkyl aryl, in an anhydrous basic medium to give compound III. 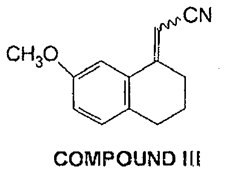
(b) reducing Compound III by the salt of an alkali metal in metallic hydride in an alcohol, hydroalcohol or THF solvent to give compound IVa. 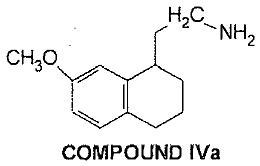
(c) acylation of Compound IVa with acetic anhydride or acetyl chloride to produce Compound Va 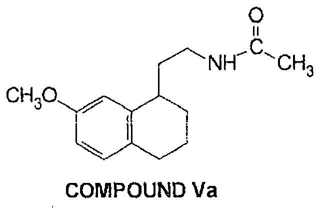
(d) oxidation (aromatization) of Compound Va, and
(e) purification to yield the desired product Agomelatine (I), with a melting point of 108-109 °C.

14. A procedure, according to claim 13, where the base to obtain compound III is NaH, Li₂O, LiOH, NaOH or potassium *tert*-butoxide, preferably LiOH, Li₂O or NaH.

15. A procedure, according to claim 13, where the metallic hydride used to obtain compound IVa is NaBH₄.

16. A procedure, according to claim 13, where the metallic hydride mentioned in claim 15 is used in the presence of salts such as AlCl₃, NiCl₂ or CoCl₂, preferably NiCl₂.

17. A procedure, according to claim 13, where the solvent used in the reduction step with the metallic hydride is an alcohol with up to 4 carbon atoms, eventually with the addition of water.

18. A procedure, according to claim 13, where the oxidizing agent in the aromatization step to obtain the Compound (I) is 2,3-dichloro-5,6-dicyano benzoquinone (DDQ).

19. A procedure for the preparation of Compound (I), according to claim 13, further comprising a purifying procedure with a crystallization solvent which is an alcohol with up to 4 carbon atoms, ethyl acetate, methyl-ethyl ketone, toluene, acetonitrile or mixtures thereof, eventually with the addition of water.

## Patentansprüche

1. Verfahren für die Herstellung von N-[2-(7-Methoxy-1-naphthyl)ethyl]acetamid, Verbindung (I) 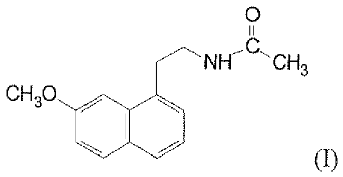 **dadurch gekennzeichnet, dass** Verbindung (II) umgesetzt wird mit einem Ester der Cyanomethylphosphonsäure [(RO)₂P(O)CH₂CN, wobei R = Alkyl, Aryl, Alkylarly] in einem wasserfreien basischen Medium, um Verbindung (III) zu ergeben; 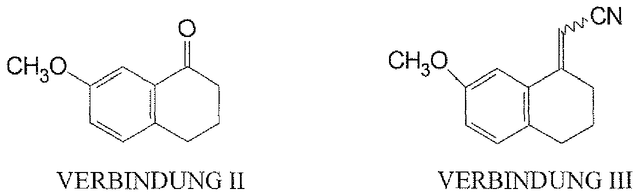 wobei die Verbindung (III) dann umgewandelt wird in Verbindung (IV), oder ein pharmazeutisch annehmbares Salz derselben, durch Reduktion mit einem Metallsalz eines Metallhydrids in einem aprotischen Lösungsmittel, und ihrer anschließenden Isomerisierung mit einer protischen Säure; 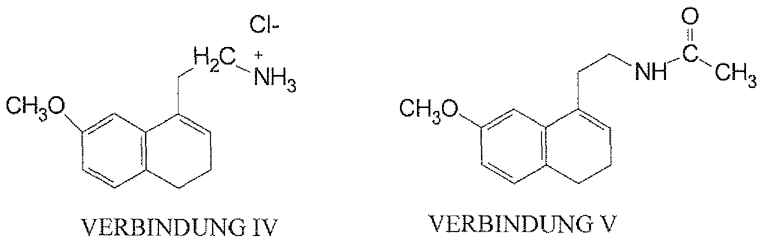 wobei diese Umsetzung gefolgt wird durch einen Acylierungsprozess, um Verbindung (V) zu erhalten; und schließlich durch Oxidation (Aromatisierung) von Verbindung (V), um N-[(7-Methoxy-1-naphthyl)ethyl]acetamid, Verbindung (I), zu erhalten.

2. Verfahren nach Anspruch 1, wobei die Base für den Erhalt der Verbindung (III) NaH, Li₂O, LiOH, NaOH, KOH oder Kalium-*tert*-butoxid ist, bevorzugt Kaliumhydroxid (KOH).

3. Verfahren nach Anspruch 1, wobei das Lösungsmittel für den Erhalt der Verbindung (III) eine Etherverbindung ist, wie Diisopropylether, Tetrahydrofuran oder Dimethylsulfoxid, bevorzugt Dimethylsulfoxid (DMSO).

4. Verfahren nach Anspruch 1, **dadurch gekennzeichnet, dass** das für den Erhalt der Verbindung (VI) verwendete Metallhydrid NaBH₄ oder LiAlH₄ ist, bevorzugt LiAlH₄.

5. Verfahren nach Anspruch 1 und 4, **dadurch gekennzeichnet, dass** das Metallhydrid in der Gegenwart einer Lewissäure verwendet wird, die ausgewählt ist aus der Gruppe bestehend aus AlCl₃, NiCl₂ oder CoCl₂, bevorzugt AlCl₃.

6. Verfahren nach Anspruch 1, **dadurch gekennzeichnet, dass** das für die Reduktion mit dem Metallhydrid verwendete aprotische Lösungsmittel eine Etherverbindung, wie Diisopropylether, Tetrahydrofuran oder Mischungen derselben mit Toluol ist.

7. Verfahren nach Anspruch 1, **dadurch gekennzeichnet, dass** die für die Isomerisierung und den Erhalt der Verbindung (IV) verwendete protische Säure eine wässrige Säure ist, wie HCl, H₂SO₄ oder HBr, bevorzugt HCl.

8. Verfahren nach Anspruch 1, **dadurch gekennzeichnet, dass** das Acylierungsmittel, das für den Erhalt der Verbindung (V) verwendet wird, Essigsäureanhydrid/Natriumacetat ist.

9. Verfahren nach Anspruch 1, **dadurch gekennzeichnet, dass** das Acylierungsmittel, das für den Erhalt der Verbindung (V) verwendet wird, Acetylchlorid in der Gegenwart oder Abwesenheit einer Base, wie Triethylamin, Pyridin, Kaliumcarbonat, Natriumacetat oder dergleichen, ist.

10. Verfahren nach Anspruch 1, **dadurch gekennzeichnet, dass** das Oxidationsmittel in dem abschließenden Schritt der Aromatisierung für den Erhalt von N-[2-(7-Methoxy-1-naphthyl)ethyl]acetamid, Verbindung (I), 2,3-Dichlor-5,6-dicyanobenzochinon (DDQ) ist.

11. Verfahren nach Anspruch 1, **dadurch gekennzeichnet, dass** die abschließende Oxidation für den Erhalt von N-[2-(7-Methoxy-1-naphthyl)ethyl]acetamid, Verbindung (I), durch Katalyse mit Pd/C durchgeführt wird, ohne weitere Zugabe von Cokatalysatoren, in einem Alkyl- oder Arylkohlenwasserstoff.

12. Verfahren für die Reinigung von Verbindung (I), nach Anspruch 1, wobei das Kristallisationslösungsmittel ein Alkohol mit bis zu vier Kohlenstoffatomen, Ethylacetat, Methyl-Ethyl-Keton, Aceton, Isopropylether, Toluol, Acetonitril oder Mischungen derselben, mit der optionalen Zugabe von Wasser ist.

13. Verfahren zur Herstellung von N-[(7-Methoxy-1-naphthyl)ethyl]acetamid (Agomelatin) (Verbindung I) umfassend:
(a) Umsetzen von Verbindung II 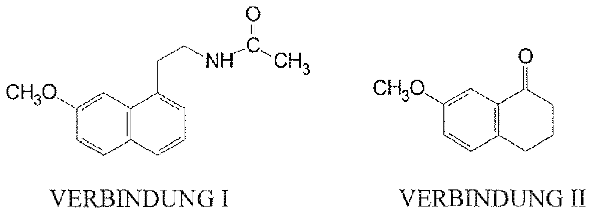 mit (RO)₂P(O)CH₂CN, wobei R = Alkyl, Aryl, Alkylaryl, in einem wasserfreien basischen Medium, um Verbindung III zu ergeben. 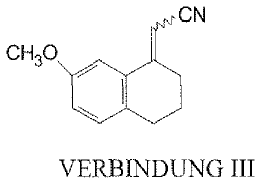
(b) Reduzieren von Verbindung III durch das Salz eines Alkalimetalls in einem metallischen Hydrid in einem Alkohol-, Hydroalkohol- oder THF-Lösungsmittel, um Verbindung IVa zu ergeben. 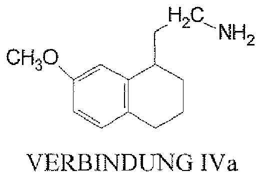
(c) Acylierung von Verbindung IVa mit Essigsäureanhydrid oder Acetylchlorid, um Verbindung Va herzustellen. 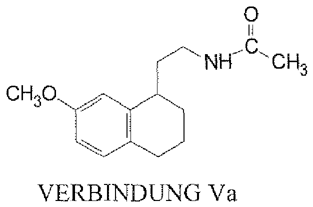
(d) Oxidation (Aromatisierung) von Verbindung Va, und
(e) Reinigung, um das gewünschte Produkt Agomelatin (I) mit einem Schmelzpunkt von 108-109°C zu erhalten.

14. Verfahren nach Anspruch 13, wobei die Base, um Verbindung III zu erhalten, NaH, Li₂O, LiOH, NaOH oder Kalium-*tert*-butoxid ist, bevorzugt LiOH, Li₂O oder NaH.

15. Verfahren nach Anspruch 13, wobei das metallische Hydrid, um Verbindung IVa zu erhalten, NaBH₄ ist.

16. Verfahren nach Anspruch 13, wobei das metallische Hydrid, das in Anspruch 15 erwähnt wird, in der Gegenwart von Salzen verwendet wird, wie AlCl₃, NiCl₂ oder CoCl₂, bevorzugt NiCl₂.

17. Verfahren nach Anspruch 13, wobei das in dem Reduktionsschritt mit dem metallischen Hydrid verwendete Lösungsmittel ein Alkohol mit bis zu vier Kohlenstoffatomen ist, eventuell mit der Zugabe von Wasser.

18. Verfahren nach Anspruch 13, wobei das Oxidationsmittel in dem Aromatisierungsschritt, um die Verbindung (I) zu erhalten, 2,3-Dichlor-5,6-dicyanobenzochinon (DDQ) ist.

19. Verfahren für die Herstellung von Verbindung (I) nach Anspruch 13, weiter umfassend ein Reinigungsverfahren mit einem Kristallisationslösungsmittel, welches ein Alkohol mit bis zu vier Kohlenstoffatomen, Ethylacetat, Methyl-Ethyl-Keton, Toluol, Acetonitril oder Mischungen derselben ist, eventuell mit der Zugabe von Wasser.

## Revendications

1. Procédé de préparation de N-[2-(7-méthoxy-1-naphtyl)éthyl]acétamide, composé (I) 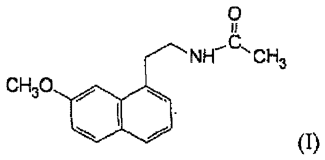 **caractérisé en ce que** le composé (II) est mis à réagir avec un ester de l'acide cyanométhylphosphonique [(RO)₂P(O)CH₂CN, dans lequel R = alkyle, aryle, alkylaryle] dans un milieu basique anhydre pour donner le composé (III) ; 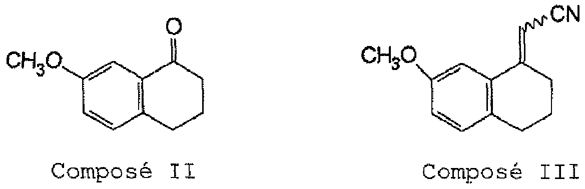 le composé (III) est ensuite transformé en composé (IV), ou en un sel pharmaceutiquement acceptable de celui-ci, par le biais d'une réduction avec un sel métallique d'un hydrure de métal dans un solvant aprotique, et une isomérisation ultérieure avec un acide protique ; 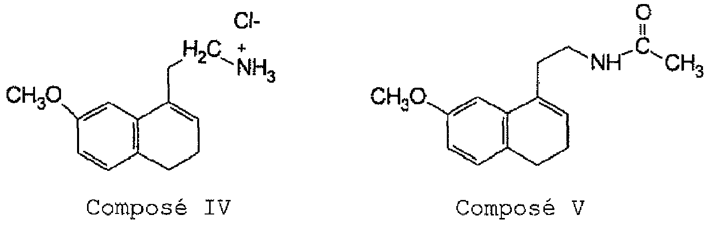 cette réaction est suivie par un procédé d'acylation pour obtenir le composé (V) ; et finalement par oxydation (aromatisation) du composé (V) pour obtenir le N-[(7-méthoxy-1-naphtyl)éthyl]acétamide, composé (I).

2. Procédé selon la revendication 1, dans lequel la base pour l'obtention du composé (III) est NaH, Li₂O, LiOH, NaOH, KOH ou du *tert*-butoxyde de potassium, de préférence de l'hydroxyde de potassium (KOH).

3. Procédé selon la revendication 1, dans lequel le solvant pour l'obtention du composé (III) est un composé éthéré tel que l'éther diisopropylique, le tétrahydrofurane le diméthylsulfoxyde, de préférence le diméthylsulfoxyde (DMSO).

4. Procédé selon la revendication 1, **caractérisé en ce que** l'hydrure de métal utilisé pour l'obtention du composé (IV) est NaBH₄ ou LiAlH₄, de préférence LiAlH₄.

5. Procédé selon la revendication 1 et 4, **caractérisé en ce que** l'hydrure de métal utilisé en présence d'un acide de Lewis est choisi dans le groupe constitué par AlCl₃, NiCl₂ ou CoCl₂, de préférence AlCl₃.

6. Procédé selon la revendication 1, **caractérisé en ce que** le solvant aprotique utilisé pour la réduction avec l'hydrure de métal est un composé éthéré tel que l'éther diisopropylique, le tétrahydrofurane ou des mélanges de ceux-ci avec du toluène.

7. Procédé selon la revendication 1, **caractérisé en ce que** l'acide protique utilisé pour l'isomérisation et l'obtention du composé (IV) est un acide aqueux tel que HCl, H₂SO₄ ou HBr, de préférence HCl.

8. Procédé selon la revendication 1, **caractérisé en ce que** l'agent d'acylation utilisé pour l'obtention du composé (V) est un mélange d'anhydride acétique/acétate de sodium.

9. Procédé selon la revendication 1, **caractérisé en ce que** l'agent d'acylation utilisé pour l'obtention du composé (V) est le chlorure d'acétyle en présence ou en l'absence d'une base telle que la triéthylamine, la pyridine, le carbonate de potassium, l'acétate de sodium ou analogues.

10. Procédé selon la revendication 1, **caractérisé en ce que** l'agent d'oxydation dans l'étape finale d'aromatisation pour l'obtention de N-[2-(7-méthoxy-1-naphtyl)éthyl]acétamide, composé (I), est la 2,3-dichloro-5,6-dicyanobenzoquinone (DDQ).

11. Procédé selon la revendication 1, **caractérisé en ce que** l'oxydation finale pour l'obtention de N-[2-(7-méthoxy-1-naphtyl)éthyl]acétamide, composé (I), est réalisée par catalyse avec Pd/C, sans autre addition de co-catalyseurs, dans un hydrocarbure alkylique ou arylique.

12. Procédé de purification du composé (I) selon la revendication 1, dans lequel le solvant de cristallisation est un alcool comprenant jusqu'à 4 atomes de carbone, l'acétate d'éthyle, la méthyléthylcétone, l'acétone, l'éther isopropylique, le toluène, l'acétonitrile ou des mélanges de ceux-ci, avec l'addition facultative d'eau.

13. Procédé de préparation de N-[(7-méthoxy-1-naphtyl)éthyl]acétamide (Agomélatine) (Composé I) comprenant :
(a) faire réagir le composé (II) 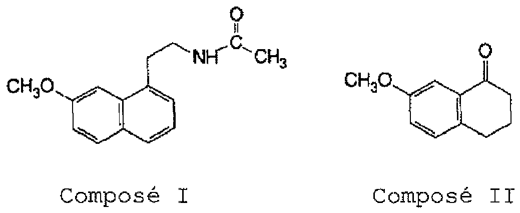 avec (RO)₂P(O)CH₂CN, dans lequel R = alkyle, aryle, alkylaryle, dans un milieu basique anhydre pour donner le composé (III) 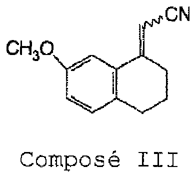
(b) réduire le composé (III) par le sel d'un métal alcalin dans un hydrure de métal dans un alcool, un hydroalcool ou un solvant à base de THF pour donner le composé (IVa) 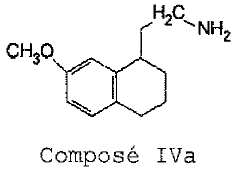
(c) acyler le composé (IVa) avec de l'anhydride acétique ou du chlorure d'acétyle pour produire le composé (Va) 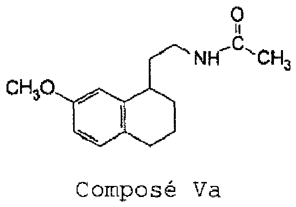
(d) oxyder (aromatiser) le composé (Va), et
(e) purifier pour donner le produit souhaité, Agomélatine (I), avec un point de fusion de 108 à 109 °C.

14. Procédé selon la revendication 13, dans lequel la base pour obtenir le composé (III) est NaH, Li₂O, LiOH, NaOH ou le *tert*-butoxyde de potassium, de préférence LiOH, Li₂O ou NaH.

15. Procédé selon la revendication 13, dans lequel l'hydrure de métal utilisé pour obtenir le composé (IVa) est NaBH₄.

16. Procédé selon la revendication 13, dans lequel l'hydrure de métal mentionné selon la revendication 15 est utilisé en présence de sels tels que AlCl₃, NiCl₂ ou CoCl₂, de préférence NiCl₂.

17. Procédé selon la revendication 13, dans lequel le solvant utilisé dans l'étape de réduction avec l'hydrure de métal est un alcool comprenant jusqu'à 4 atomes de carbone, éventuellement avec l'addition

18. Procédé selon la revendication 13, dans lequel l'agent d'oxydation dans l'étape d'aromatisation pour obtenir le composé (I) est la 2,3-dichloro-5,6-dicyanobenzoquinone (DDQ).

19. P.rocédé de préparation du composé (I) selon la revendication 13, comprenant en outre un procédé de purification avec un solvant de cristallisation qui est un alcool comprenant jusqu'à 4 atomes de carbone, l'acétate d'éthyle, la méthyléthylcétone, le toluène, l'acétonitrile ou des mélanges de ceux-ci, éventuellement avec l'addition d'eau.
